# EUROPÄISCHE PATENTANMELDUNG

(11) **EP 4 729 016 A1**
(43) Veröffentlichungstag der Anmeldung: **22.04.2026**
(21) Anmeldenummer: 25209550.0
(22) Anmeldetag: 17.10.2025
(51) Int. Cl.: A61B 50/34, A61B 50/33

(54) **SIEBKORB, VERFAHREN ZUM GREIFEN EINES SIEBKORBS UND SYSTEM**

(30) Priorität: 18.10.2024 DE 102024130348
(71) Anmelder: Aesculap AG, 78532 Tuttlingen (DE)
(72) Erfinder: Jansen-Troy, Arne, 78333 Stockach (DE); Römpp, Leo, 78739 Hardt (DE); Hermann, Johannes, 72355 Schömberg (DE); Koller, Tobias, 78532 Tuttlingen (DE)
(74) Vertreter: DREISS Patentanwälte PartG mbB

(57) **Zusammenfassung**

Die Erfindung betrifft ein Siebkorb (9) mit einem Siebkorbpositionierelement (1) zum Positionieren und Erleichtern des Greifens des Siebkorbs (9), der einen Lagerraum (21) zum Spülen von Sterilgut und einen davon mittels einer Trennwand (12) getrennten Schutzraum (11) aufweist, innerhalb dessen ein Greifer (25) eintauchen und ein Abstützteil des Siebkorbs (9) hintergreifen kann.

## Beschreibung

Die Erfindung betrifft gemäß Anspruch 1 einen Siebkorb mit einem Siebkorbpositionierelement. Ferner betrifft die Erfindung ein System, das den Siebkorb und das Siebkorbpositionierelement umfasst und ein Verfahren zum Greifen des Siebkorbs.

In der DE 10 2020 131 104 A1 ist eine Sterilisiersiebschale mit Fixiergriff offenbart, welche zum Einsetzen in und Entnehmen aus einer Containerwanne vorgesehen und ausgebildet ist.

DE 10 2020 103 131 A1 offenbart ein Kennzeichnungssystem für Sterilcontainer und Siebschalen.

DE 10 2018 104 942 A1 offenbart eine Sterilisiersiebschale mit einem mittels einer Betätigung in die Entnahmeposition überführbaren Griff.

Aufgabe der Erfindung ist es, einen Siebkorb zu schaffen, der mittels eines Greifers insbesondere automatisiert gegriffen werden kann.

Die Erfindung betrifft gemäß Patentanspruch 1 einen Siebkorb mit einem Siebkorbpositionierelement zum Positionieren und Erleichtern des Greifens des Siebkorbs, der einen Lagerraum zum Spülen von Sterilgut und einen davon mittels einer Trennwand getrennten Schutzraum aufweist, innerhalb dessen ein Greifer eintauchen und ein Abstützteil des Siebkorbs hintergreifen kann.

Das Siebkorbpositionierelement dient mechanisch der genaueren Positionierung bzw. starken Einschränkung der Bewegung eines Siebkorbes in einem Sterilgutbehälter, der auch als Sterilgut-Container bezeichnet wird. Dabei kann das Siebkorbpositionierelement formschlüssig und/oder kraftschlüssig auf einen freien Randabschnitt des Siebkorbs aufgesteckt sein. Das Positionierelement dient außerdem dem Tracking und Tracing mithilfe von optischen bzw. maschinenlesbaren Markierungen zur Navigation und einem Informationsabgleich mit hinterlegten Daten.

Der Schutzraum verhindert, dass ein Instrument oder ein Teil davon an einer Stelle des Siebkorbs liegt, an der der Greifer eintaucht. An eine solche Stelle könnte ein Instrument nämlich insbesondere dann gelangen, wenn die Instrumente im Siebkorb chaotisch gelagert werden oder wenn sich die Instrumente während des Transports verschieben. Es gibt zwar bestimmte Siebkorbkonfigurationen, bei denen manchen oder allen Instrumenten eine definierte Lagerungsposition bzw. ein bestimmter Lagerplatz zugeordnet ist und bei denen die so gelagerten Instrumente relativ sicher gegen eine unbeabsichtigte Verlagerung gehalten bzw. gesichert sind. Ein Großteil der Siebkörbe weist aber nicht für alle aufgenommenen Instrumente oder sogar für kein aufgenommenes Instrument eine spezifische Lagerungsposition auf. In diesen Fällen sind die Instrumente im Prinzip wie Schüttgut im Bulk in dem Siebkorb abgelegt, ohne dass diese eine definierte Position oder Lage einnehmen und dementsprechend auch nicht gegen eine unbeabsichtigte Verlagerung gesichert sind. Jedenfalls verhindert der Schutzraum beim Eintauchen bzw. Einfahren des Greifers in den Siebkorb, dass der Greifer mit Instrumenten kollidiert. Somit verhindert der Schutzraum, dass die Instrumente, der Siebkorb oder ein automatisiertes, insbesondere robotisches Greifsystem des Greifers beschädigt wird. Überdies wird durch den Schutzraum verhindert, dass Schmutz oder Verbrauchsartikel in den Arbeitsbereich des Greifer gelangen können.

Der Schutzraum ist vorzugsweise oben offen, bietet aber einen Schutz zur unteren Seite und weiteren mindestens drei Seiten, welche nicht bereits durch den Rand des Siebkorbs abdeckt sind. Der Schutzraum ist vorzugsweise nur so groß, dass der Greifer von oben einfahren kann und sich durch eine horizontale Bewegung im Siebkorb unter die Abstützung bewegt und den Siebkorb herausheben kann.

Bei einer Ausführungsform ist vorgesehen, dass das Siebkorbpositionierelement vorrangig an einem Randabschnitt des Siebkorbs befestigt, insbesondere aufgesteckt ist. Der Schutzraum kann somit so gestaltet sein, dass das Siebkorbpositionierelement ohne Werkzeuge am Siebkorb befestigt werden kann. Dabei kann das Siebkorbpositionierelement insbesondere aus Kunststoff, insbesondere als Spritzgussteil ausgebildet sein.

Das Greifen des Siebkorbs kann insbesondere mittels eines Roboters erfolgen, dessen Greifer des Greifsystems gebogene Lagerösen hintergreift bzw. untergreift. Diese Lagerösen können an der Innenseite des Siebkorbs angeordnet sein und die freien Enden eines aus einem Rundstahl gebogenen Griffbügels umschließen und drehend lagern. Insofern kann bei einer Ausführungsform vorgesehen sein, dass das Abstützteil des Siebkorbs die beiden Lagerösen umfasst, in denen die Enden des gemeinsamen Griffbügels des Siebkorbs schwenkbar aufgenommen sind.

Bei einer Ausführungsform ist vorgesehen, dass die Trennwand untere Wandteile aufweist, die bezüglich einer waagerechten Spüllage des Siebkorbs jeweils
- unterhalb von Unterkanten der Lagerösen angeordnet sind, und
- in vom Randabschnitt weg und nach innen weisenden horizontalen Richtungen derart von Außenkontouren der Lagerösen beabstandet sind, dass das Siebkorbpositionierelement von oben auf den Randabschnitt aufgesteckt werden kann, ohne dass die unteren Wandteile dabei in Kontakt mit dem Lagerösen kommen.

Mit anderen Worten gesagt, kann das Siebkorbpositionierelement von oben in einer geraden Bewegung auf dem Siebkorb befestigt werden, ohne dass ein Teil der Trennwand aufgebogen werden muss.

Bei einer Ausführungsform ist vorgesehen, dass die Trennwand Zwischen-Trennwandteile aufweist, die zwischen den Lagerösen angeordnet sind.

Bei einer Ausführungsform ist vorgesehen, dass Zwischen-Trennwandteile mittels einer Verbindungswand miteinander verbunden sind.

Bei einer Ausführungsform ist vorgesehen, dass die Trennwand Innen-Trennwandteile aufweist, die bezüglich einer waagerechten Spüllage des Siebkorbs in einer vom Randabschnitt senkrecht weg und zum Lagerraum hin weisenden horizontalen Richtung so weit von den Lagerösen beabstandet sind, dass horizontale Arme des Greifers oder der Greifer bis unterhalb von Unterkanten der Lagerösen eintauchen können.

Bei einer Ausführungsform ist vorgesehen, dass die Innen-Trennwandteile Aussparungen aufweisen, in denen der Griffbügel teilweise aufgenommen und am Innen-Trennwandteil abgestützt ist, wenn sich der Griffbügel in einer waagerechten Lage befindet.

Bei einer Ausführungsform ist vorgesehen, dass die Trennwand zumindest ein Lateral-Verbindungsteil aufweist, das eines der Innen-Trennwandteile mit einer von mehreren Flächen verbindet, auf denen ein weiterer Siebkorb absetzbar ist.

Bei einer Ausführungsform ist vorgesehen, dass das untere Wandteil eine maschinenlesbare Markierung aufweist, die insbesondere als Muster ausgeführt ist. Eine solche maschinenlesbare Markierung ist insbesondere optisch erkennbar, beispielsweise mittels einer Kamera. Eine solche maschinenlesbare Markierung ist vorzugsweise an der unteren Stelle des Schutzraums in horizontaler Position angebracht, sodass diese Markierung in einer Draufsicht sichtbar ist. Diese Markierung ermöglicht es beispielsweise dem Greifsystem, mithilfe einer Roboterführungskamera redundant zu prüfen, ob sich Objekte im Arbeits- bzw. Verfahrweg des Greifers befinden. Diese Markierung kann eindeutig als ein definiertes Muster ausgebildet sein. Falls das Muster nicht vollständig erfasst wird, weil sich ein Objekt im Arbeitsraum befindet, fährt das automatisierte Greifsystem eine Greifposition nicht an und meldet einen Fehler über eine Schnittstelle an eine Steuerungseinrichtung. Diese maschinenlesbaren Markierungen können gedruckt oder aber bereits durch einen Spritzgussprozess in die Oberfläche des unteren Wandteils eingebracht oder auf diese aufgebracht werden.

Bei einer weiteren Ausführungsform ist vorgesehen, dass außerhalb des Schutzraums eine maschinenlesbare Schnittstelle zur Erkennung einer Position des Siebkorbs vorgesehen ist.

Die Erfindung betrifft ferner ein Verfahren zum Greifen des Siebkorbs innerhalb eines Behälters, wobei ein Arm des Greifers von oben in den Schutzraum und unter das Abstützteil bewegt wird und anschließend zum Herausheben des Siebkorbs angehoben wird.

Bei einer Ausführungsform ist das Verfahren derart ausgeführt, dass der Greifer zunächst vertikal nach unten und anschließend horizontal unter das Abstützteil bewegt wird.

Bei einer Ausführungsform ist das Verfahren derart ausgeführt, dass von einer mit dem Greifer gekoppelten optischen

Erfassungseinrichtung ein Bereich innerhalb des Schutzraumes erfasst wird, in dem sich eine maschinenlesbare Markierung befindet, wobei ein Fehler an eine Steuerungseinrichtung gemeldet wird, wenn die maschinenlesbare Markierung nicht oder nicht ausreichend erfasst werden kann.

Bei einer Ausführungsform ist das Verfahren derart ausgeführt, dass die Steuerungseinrichtung den Greifer nach der Meldung des Fehlers veranlasst,
- sich nicht in den Schutzraum zu bewegen oder
- eine Bewegung in Richtung auf den Schutzraum zu stoppen oder
- sich wieder aus dem Schutzraum herauszubewegen.

Die Erfindung betrifft ferner ein System, das den Siebkorb und das Siebkorbpositionierelement umfasst, wobei der Behälter eine Wanne zur Aufnahme des Siebkorbs umfasst, wobei der Siebkorb mit dem Siebkorbpositionierelement in die Wanne des Behälters einsetzbar ist und derart angepasst ist, dass die mechanische Schnittstelle die Bewegung des Siebkorbs einschränkt.

Vorzugsweise weist die Vorrichtung eine Fläche auf, welche die maschinenlesbare Schnittstelle zur Erkennung des Siebkorbs und/oder die maschinenlesbare Schnittstelle zur Erkennung der Position des Siebkorbs umfasst.

In einer Ausführungsform ist die maschinenlesbare Schnittstelle zur Erkennung des Siebkorbs und/oder die maschinenlesbare Schnittstelle zur Erkennung der Position des Siebkorbs und/oder Orientierung in die Fläche eingeprägt oder auf die Fläche aufgedruckt oder anderweitig an dieser angebracht. Diese Vorrichtung ist besonders einfach in Masse fertigbar.

In einer Ausführungsform ist die Fläche zum Stapeln eines weiteren Siebkorbs ausgebildet. Dadurch sind mehrere Siebkörbe übereinander anordbar.

Vorzugsweise umfasst die maschinenlesbare Schnittstelle zur Erkennung des Siebkorbs einen optisch lesbaren Marker, insbesondere einen Barcode, einen Datamatrixcode, alphanummerische Daten oder einen QR Code, der eine Typenidentifikation des Siebkorbs maschinenlesbar codiert.

In einer Ausführungsform umfasst die maschinenlesbare Schnittstelle zur Erkennung der Position und/oder Orientierung ein Symbol mit einer eindeutigen Angabe einer Orientierung des Symbols. Dies ermöglicht eine unmissverständliche Anzeige der Orientierung.

Es kann vorgesehen sein, dass das Siebkorbpositionierelement zumindest teilweise aus Kunststoff oder zumindest teilweise aus Metall gefertigt ist.

Es kann vorgesehen sein, dass das Siebkorbpositionierelement einen Sender umfasst, der ausgebildet ist, ein elektromagnetisches Signal, insbesondere ein Bluetooth Low Energy Signal oder ein Radio Frequency Identification Signal, zur Erkennung des Siebkorbs zu senden, wobei die maschinenlesbare Schnittstelle zur Erkennung des Siebkorbs den Sender umfasst und/oder dass die Vorrichtung einen Sender umfasst, der ausgebildet ist, ein elektromagnetisches Signal, insbesondere ein Bluetooth Low Energy Signal oder ein Radio Frequency Identification Signal, zur Erkennung der Position und/oder Orientierung des Siebkorbs zu senden, wobei die maschinenlesbare Schnittstelle zur Erkennung der Position und/oder Orientierung den Sender umfasst.

Es kann vorgesehen sein, dass der Sender an das Siebkorbpositionierelement geklebt und oder kraft- oder formschlüssig fixiert ist oder dass das Siebkorbpositionierelement zumindest teilweise aus Kunststoff gefertigt ist, wobei der Sender vom Kunststoff umgeben ist.

Weitere vorteilhafte Ausführungsformen sind der folgenden Beschreibung und der Zeichnung entnehmbar. In der Zeichnung zeigt:
- Figur 1: eine perspektivische Darstellung eines Siebkorbpositionierelements,
- Figur 2: eine Aufsicht auf einen Siebkorb mit einem Siebkorbpositionierelement und einem Greifer,
- Figur 3a: Ausschnitt des Siebkorbs im Detail B von Figur 2,
- Figur 3b: eine geschnittene Ansicht entlang der gestuften Linie A-A von Figur 2,
- Figur 4: eine perspektivische Darstellung des Siebkorbs mit dem Siebkorbpositionierelement und dem Greifer, und
- Figur 5: eine perspektivische Darstellung eines Siebkorbs mit zwei Siebkorbpositionierelementen eingesetzt in einen Sterilgutbehälter.

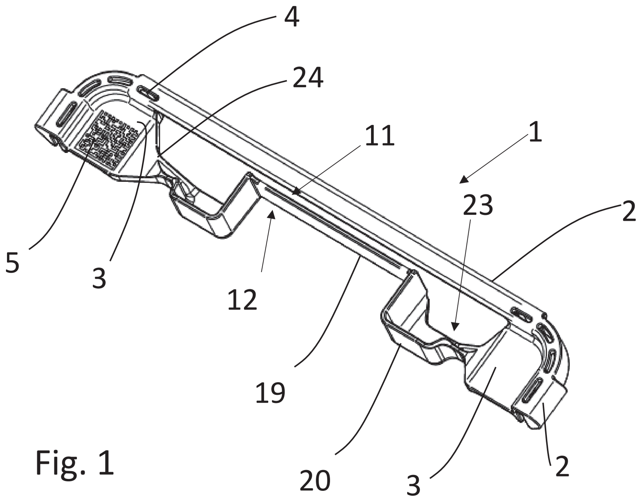

In Figur 1 ist ein Siebkorbpositionierelement 1 dargestellt. Das Siebkorbpositionierelement 1 ist zur Positionierung eines Siebkorbs in einem Behälter vorgesehen. Ein solcher Siebkorb weist vorzugsweise zwei Siebkorbpositionierelemente 1 auf. Der Behälter ist im Beispiel ein Sterilgutbehälter.

Nachfolgende Bezeichnungen wie "oben", "unten" beziehen sich auf eine waagerechte Spüllage des Siebkorbs, d.h. eine Lage, in der Instrumente innerhalb des Siebkorbs mit einer Sterilisationsflüssigkeit gespült werden, wobei die Oberfläche der Sterilisationsflüssigkeit parallel zu einer Oberkante des Siebkorbs und des Sterilgutbehälters ist.

Nachfolgende Bezeichnungen wie "außen", "innen" beziehen sich auf den Siebkorb. Das gilt insbesondere für Richtungsangaben "nach innen" und "nach außen", die Richtungen in der Waagerechten auf den Siebkorb hin bzw. vom Siebkorb weg angeben.

Bei den Instrumenten kann es sich insbesondere um chirurgische Instrumente und sonstiges medizinisches Equipment handeln.

Das Siebkorbpositionierelement 1 ist am Siebkorb anordbar. Das Siebkorbpositionierelement 1 ist im Beispiel an einem oberen Randabschnitt des Siebkorbs anordbar.

Es kann vorgesehen sein, dass das Siebkorbpositionierelement stattdessen einstückig mit dem Siebkorb ausgeführt ist.

Das Siebkorbpositionierelement 1 kann in Kunststoff oder in Metall ausgeführt sein. Es kann vorgesehen sein, dass das Siebkorbpositionierelement 1 teilweise in Kunststoff und/oder teilweise in Metall ausgeführt ist. Vorzugsweise ist das Siebkorbpositionierelement 1 ein Spritzgussteil aus Kunststoff.

Das Siebkorbpositionierelement 1 weist eine erste mechanische Schnittstelle 2 auf, welche zur Einschränkung einer Bewegung des Siebkorbs im Sterilgutbehälter ausgebildet ist. Die erste mechanische Schnittstelle 2 ist eine Schnittstelle zum Sterilgutbehälter. Die erste mechanische Schnittstelle 2 umfasst im Beispiel Anlageflächen zur Reduzierung eines Spiels zwischen Siebkorb und Sterilgutbehälter. Die Anlageflächen sind im Beispiel an wenigstens einem äußeren Randbereich des Siebkorbpositionierelementes 1 angeordnet. Im Beispiel sind drei nach außen gerichtete Anlageflächen vorgesehen. Es kann vorgesehen sein, dass die erste mechanische Schnittstelle 2 eine, zwei oder mehr als drei Anlageflächen umfasst.

Das Siebkorbpositionierelement 1 umfasst außerdem zwei nach oben gerichtete Flächen 3. Diese Flächen 3 können zum Stapeln des Siebkorbs mit einem weiteren Siebkorb vorgesehen sein. Die Siebkörbe können unterschiedlich hoch sein. Der obere Siebkorb kann zum Stapeln auf den am unteren Siebkorb vorgesehenen Flächen aufsetzen.

Das Siebkorbpositionierelement 1 weist im Beispiel zwei Eckstücke und einen Steg auf, der die zwei Eckstücke miteinander verbindet.

In einer Ausführungsform weist das Siebkorbpositionierelement 1 wenigstens einen Spülkanal 4 auf, der durch das Siebkorbpositionierelement 1 hindurchführt. Im Beispiel sind je Eckstück vier voneinander beabstandete Spülkanäle vorgesehen. Die Spülkanäle sind im Beispiel als Langloch ausgebildet. Die Spülkanäle können auch eine andere Form oder Position aufweisen. Es können auch mehr oder weniger als vier Spülkanäle vorgesehen sein.

Eine erste maschinenlesbare Schnittstelle 5 ist im Beispiel auf zumindest eine der Flächen 3 aufgedruckt. Es kann auch vorgesehen sein, dass die erste maschinenlesbare Schnittstelle 5 in diese Fläche 3 eingeprägt ist. Außerdem kann die maschinenlesbare Schnittstelle 5 bei einem Spritzgussvorgang des Siebkorbpositionierelement 1 erzeugt werden.

Die erste maschinenlesbare Schnittstelle 5 umfasst in einer Ausführungsform einen optisch lesbareren Marker, der zumindest einen Typ des Siebkorbs maschinenlesbar codiert. Der optisch lesbare Marker ist z.B. ein Barcode oder ein Datamatrixcode oder ein QR Code. Der optisch lesbare Marker umfasst in einer Ausführungsform alphanummerische Daten.

Die erste maschinenlesbare Schnittstelle 5 umfasst in einer Ausführungsform einen Sender. Der Sender ist ausgebildet, ein elektromagnetisches Signal zur Erkennung des Siebkorbs zu senden. Das elektromagnetische Signal ist z.B. ein Bluetooth Low Energy Signal oder ein Radio Frequency Identification Signal.

Der Sender kann an das Siebkorbpositionierelement 1 geklebt oder kraft- und oder formschlüssig fixiert sein. Es kann vorgesehen sein, dass das Siebkorbpositionierelement 1 zumindest teilweise aus Kunststoff gefertigt ist und der Sender vom Kunststoff umgeben ist.

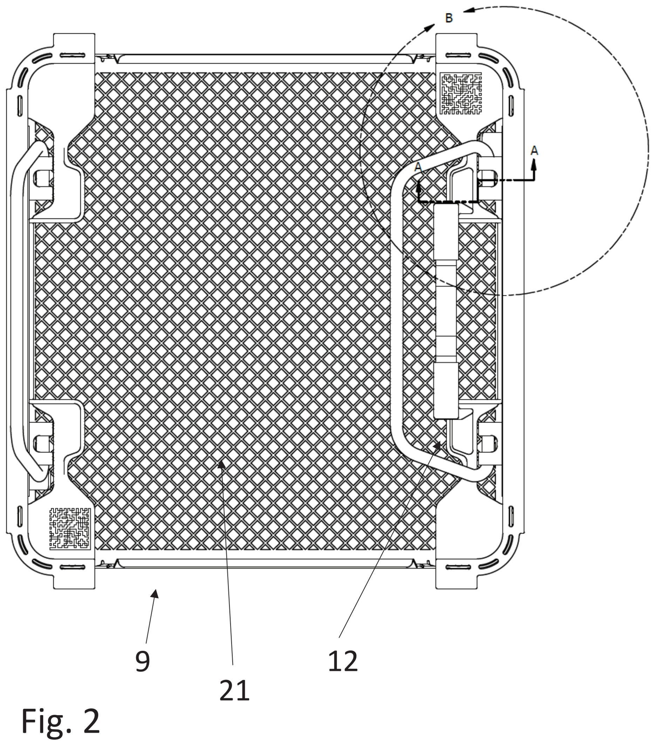

Das Siebkorbpositionierelement 1 weist einen Schutzraum 11 auf, der teilweise von einer Trennwand 12 begrenzt wird. Der Schutzraum 11 ist von einem aus Figur 2 ersichtlichen Lagerraum 21 mittels der Trennwand 12 getrennt. In dem Lagerraum 21 können zu sterilisierende Instrumente bzw. Sterilgut mit Steriliationsflüssigkeit gespült und somit sterilisiert werden.

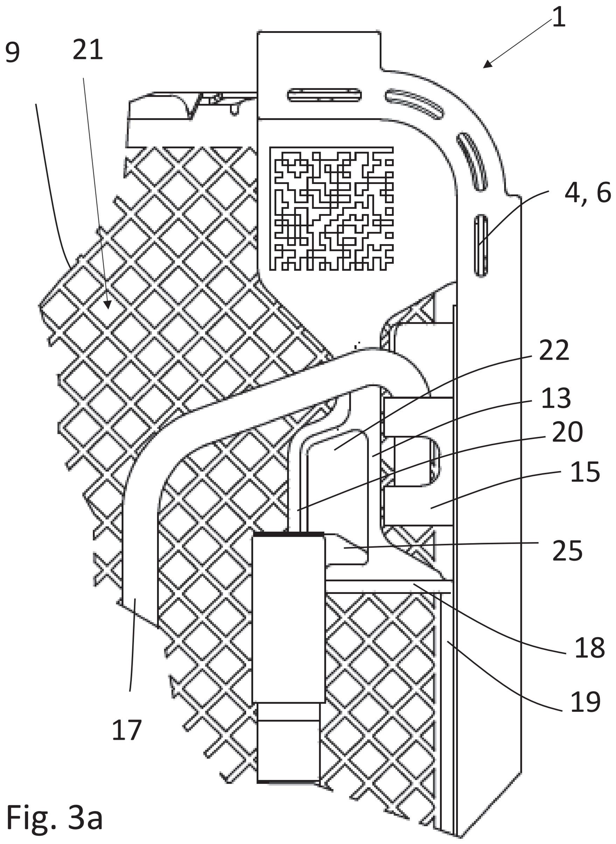

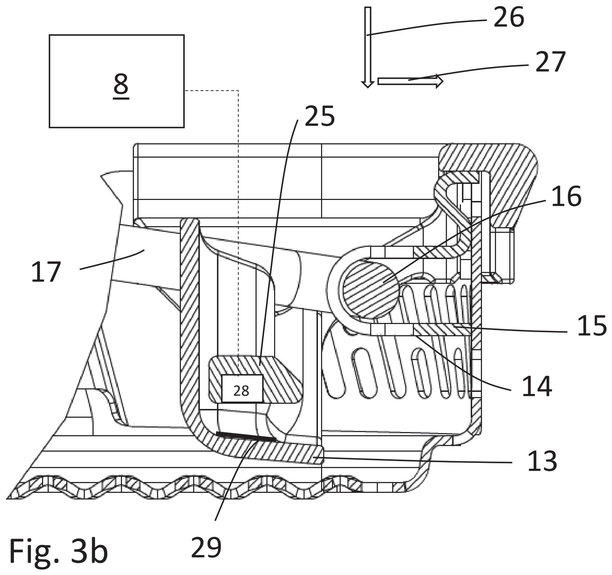

Eine mehrdimensional komplexe Formgebung der Trennwand 12 wird nachfolgend anhand einer Zusammenschau von Figuren 1 bis 3b erläutert. Figur 3b zeigt dabei ein Detail des Siebkorbs 9 mit einem der beiden Siebkorbpositionierelemente 1. Figur 3b zeigt ein Detail aus Figur 2 entlang der gestuften Linie A-A.

Die Trennwand 12 weist untere Wandteile 13 auf, die bezüglich einer waagerechten Spüllage des Siebkorbs 9 jeweils unterhalb von Unterkanten 14 von Lagerösen 15 angeordnet sind. Die jeweiligen unteren Wandteile 13 sind in vertikaler Projektion auf die Grundfläche vom Randabschnitt 6 weg und nach innen weisenden horizontalen Richtungen derart von den Außenkontouren der Lagerösen 15 beabstandet, dass das Siebkorbpositionierelement 1 auf den Randabschnitt 6 aufgesteckt werden kann, ohne dass die unteren Wandteile 13 dabei in Kontakt mit dem Lagerösen kommen.

Diese Lagerösen 15 sind aus einem Blech des Siebkorbs 9 gebogen und nehmen zwei Enden 16 von Griffbügeln 17 schwenkbar auf. Die Griffbügel 17 bestehen aus einem gebogenen Rundstahl.

Die Trennwand 12 weist ferner Zwischen-Trennwandteile 18 auf, die zwischen den Lagerösen 15 angeordnet sind. Die Zwischen-Trennwandteile 18 sind mittels einer Verbindungswand 19 miteinander verbunden.

Die Trennwand 12 weist ferner Innen-Trennwandteile 20 auf, die bezüglich einer waagerechten Spüllage des Siebkorbs 8 in einer von einem Randabschnitt 6 des Siebkorbs 9 senkrecht weg und zum Lagerraum 21 hin weisenden horizontalen Richtung so weit von den Lagerösen 15 beabstandet sind, dass horizontale Arme 22 zumindest eines Greifers 25 bis unterhalb von den Unterkanten 43 der Lagerösen 15 eintauchen können.

Die Innen-Trennwandteile 20 weisen Aussparungen 23 auf, in denen der Griffbügel 17 teilweise aufgenommen und am Innen-Trennwandteil 20 abgestützt ist, wenn sich der Griffbügel 17 in einer waagerechten Lage befindet.

Die Trennwand 12 weist ein Lateral-Verbindungsteil 24 auf, das eines der Innen-Trennwandteile 20 mit einer von mehreren Flächen 3 verbindet, auf denen ein weiterer Siebkorb absetzbar ist.

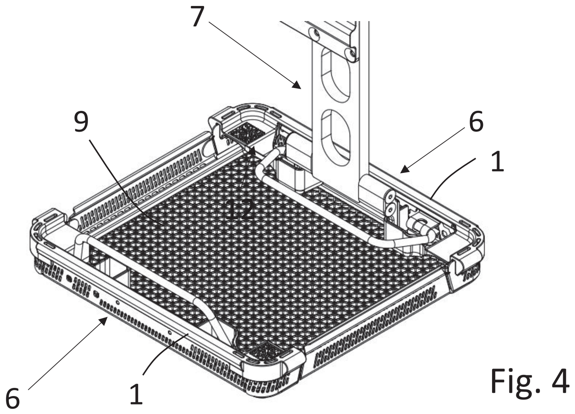

In Figur 4 ist eine perspektivische Ansicht eines exemplarischen Siebkorbs 9 mit zwei Siebkorbpositionierelementen 1 dargestellt. Im Beispiel sind zwei gleiche Siebkorbpositionierelemente 1 vorgesehen. Die Siebkorbpositionierelemente 1 sind im Beispiel an den einander gegenüberliegenden Randabschnitten 6 des Siebkorbs 9 angeordnet.

Je ein Siebkorbpositionierelement 1 ist im Beispiel an einer Seite des Siebkorbs 9 angeordnet. Die Eckstücke sind jeweils an einer Ecke des Siebkorbs 9 angeordnet.

Der Siebkorb 9 hat im Beispiel eine quadratische Grundfläche. Bei einer alternativen Ausführungsform mit ungleichen Seitenlängen und rechteckiger Grundfläche sind die Siebkorbpositionierelemente 1 vorzugsweise an den kürzeren Seiten bzw. den zugehörigen Randabschnitten befestigt.

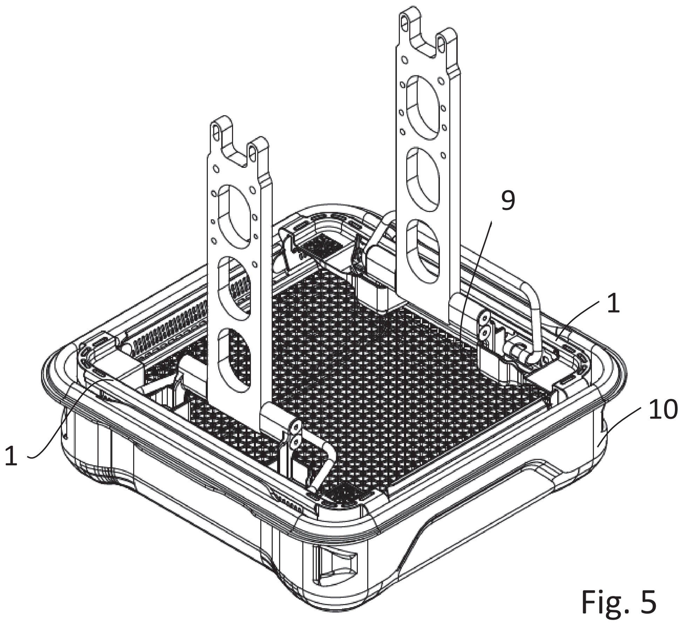

In Figur 5 ist ein System dargestellt, das den Siebkorb 9 mit den zwei Siebkorbpositionierelementen 1 und den Behälter 10 umfasst. Der Behälter 10 umfasst eine Wanne zur Aufnahme des Siebkorbs 9. Der Siebkorb 9 ist mit dem Siebkorbpositionierelement 1 in die Wanne des Behälters 10 einsetzbar.

Nachfolgend wird ein Verfahren zum Greifen des Siebkorbs 9 innerhalb des Behälters 10 erläutert. Dabei werden die Arme 22 des Greifers 25 vertikal von oben in den Schutzraum 11 hinein bewegt, wobei die Bewegungsrichtung in Figur 3b anhand eines Pfeils 26 dargestellt ist. Anschließend werden die Arme 22 des Greifers 25 horizontal unter zumindest ein Abstützteil bewegt, das im Ausführungsbeispiel von den beiden Lagerösen 15 gebildet wird. Diese Bewegungsrichtung ist in der Zeichnung anhand eines weiteren Pfeils 27 dargestellt. Dabei hintergreifen bzw. untergreifen die Arme 22 des Greifers 25 die Lagerösen 15. Die Arme 22 werden dann wieder angehoben und kommen dabei an den Unterseiten 14 der Lagerösen 15 zum anliegen, die somit ein Abstützteil für die Arme 22 bilden.

In einer kontinuierlichen Bewegung entgegen der Bewegungsrichtung des Pfeils 26 wird anschließend der Greifer 25 mit den Armen 22 zum Herausheben des Siebkorbs 9 aus dem Behälter 10 angehoben.

Ein Einsetzen des Siebkorbs 9 in den Behälter 10 erfolgt analog dazu in umgekehrter Reihenfolge und mit entgegengesetzten Bewegungsrichtungen.

Bei einer alternativen Ausführungsform des Verfahrens erfolgt die Bewegung nicht gemäß den Pfeilen 25, 27 exakt vertikal und horizontal. Stattdessen bewegen sich die Arme schräg oder bogenförmig, um den Siebkorb schneller in den Behälter einzusetzen und wieder zu entnehmen.

Überdies muss das Abstützteil nicht die Lageröse 15 sein. Insofern kann auch nur ein einziges Abstützteil und/oder ein einziger Arm vorgesehen sein.

Mit dem Greifer 25 kann eine optische Erfassungseinrichtung 28 (Figur 3b) gekoppelt sein, die einen Bereich innerhalb des Schutzraumes 11 erfasst, in dem sich eine maschinenlesbare Markierung 29 befindet. Ein Fehler wird an die Steuerungseinrichtung 8 gemeldet, wenn die maschinenlesbare Markierung 29 nicht oder nicht ausreichend erfasst werden kann. In diesem Fall wird nämlich davon ausgegangen, dass sich ein Objekt im Schutzraum befindet. Die Erfassungseinrichtung 28 ist eine Kamera und die maschinenlesbare Markierung 29 ist optisch erkennbar. Die maschinenlesbare Markierung 29 ist an der unteren Stelle des Schutzraums in einer eher horizontalen Position angebracht, sodass diese Markierung 29 in einer Draufsicht sichtbar ist. Diese Markierung 29 ermöglicht es mithilfe der Steuerungseinrichtung 8, die über eine Datenleitung und eine Schnittstelle mit der Kamera verbunden ist, zu prüfen, ob sich Objekte im Arbeits- bzw. Verfahrweg des Greifers 25 befinden. Diese Markierung 29 ist eindeutig als ein definiertes Muster ausgebildet. Falls das Muster nicht vollständig erfasst wird, weil sich ein Objekt im Arbeitsraum befindet, fährt das automatisierte Greifsystem eine Greifposition nicht an und meldet einen Fehler über eine Schnittstelle an die Steuerungseinrichtung 8. Um genau zu sein, veranlasst die Steuerungseinrichtung 8 den Greifer 25 nach der Meldung des Fehlers,
- sich nicht in den Schutzraum 11 zu bewegen oder
- eine Bewegung in Richtung auf den Schutzraum 11 zu stoppen oder
- sich wieder aus dem Schutzraum 11 herauszubewegen.

Jeder der beiden unteren Wandteile 13 weist eine eigene maschinenlesbare Markierung 29 auf, die gedruckt oder aber bereits durch einen Spritzgussprozess in die Oberflächen der unteren Wandteile 13 eingebracht wird. Alternativ kann die Markierung 29 auch zerspanend aufgebracht werden oder durch ein Laserabtragsverfahren.

Entgegen dem vorstehend erläuterten Ausführungsbeispiel können anstelle eines einzigen Schutzraums pro Siebkorbpositionierelement 1 auch zwei möglichst kleine, nicht miteinander verbundene, Schutzräume vorgesehen sein, um den Lagerraum möglichst groß zu belassen. Damit wird mehr Platz für die chirurgischen Instrumente und weiteres Equipment geboten.

## Patentansprüche

1. Siebkorb (9) mit einem Siebkorbpositionierelement (1) zum Positionieren und Erleichtern des Greifens des Siebkorbs (9), der einen Lagerraum (21) zum Spülen von Sterilgut und einen davon mittels einer Trennwand (12) getrennten Schutzraum (11) aufweist, innerhalb dessen ein Greifer (25) eintauchen und ein Abstützteil des Siebkorbs (9) hintergreifen kann.

2. Siebkorb nach Anspruch 1, **dadurch gekennzeichnet, dass** das Siebkorbpositionierelement (1) vorrangig an einem Randabschnitt (6) des Siebkorbs (9) befestigt, insbesondere aufgesteckt ist.

3. Siebkorb nach Anspruch 2, **dadurch gekennzeichnet, dass** das Abstützteil des Siebkorbs (9) zwei Lagerösen (15) umfasst, in denen Enden (16) eines gemeinsamen Griffbügels (17) des Siebkorbs (9) schwenkbar aufgenommen sind.

4. Siebkorb nach Anspruch 3, **dadurch gekennzeichnet, dass** die Trennwand (12) untere Wandteile (13) aufweist, die bezüglich einer waagerechten Spüllage des Siebkorbs (9) jeweils
- unterhalb von Unterkanten (14) der Lagerösen (15) angeordnet sind, und
- in vom Randabschnitt (6) weg und nach innen weisenden horizontalen Richtungen derart von Außenkontouren der Lagerösen (15) beabstandet sind, dass das Siebkorbpositionierelement (1) auf den Randabschnitt (6) aufgesteckt werden kann, ohne dass die unteren Wandteile (13) dabei in Kontakt mit dem Lagerösen (15) kommen.

5. Siebkorb nach einem der Ansprüche 3 oder 4, **dadurch gekennzeichnet, dass** die Trennwand (12) Zwischen-Trennwandteile (18) aufweist, die zwischen den Lagerösen (15) angeordnet sind.

6. Siebkorb nach Anspruch 5, **dadurch gekennzeichnet, dass** Zwischen-Trennwandteile (18) mittels einer Verbindungswand (19) miteinander verbunden sind.

7. Siebkorb nach einem der Ansprüche 3 bis 6, **dadurch gekennzeichnet, dass** die Trennwand (12) Innen-Trennwandteile (20) aufweist, die bezüglich einer waagerechten Spüllage des Siebkorbs (9) in einer vom Randabschnitt (6) senkrecht weg und zum Lagerraum (21) hin weisenden horizontalen Richtung so weit von den Lagerösen (15) beabstandet sind, dass horizontale Arme (22) des Greifers (25) oder der Greifer (25) bis unterhalb von Unterkanten (14) der Lagerösen (15) eintauchen können.

8. Siebkorb nach Anspruch 7, **dadurch gekennzeichnet, dass** die Innen-Trennwandteile (20) Aussparungen (23) aufweisen, in denen der Griffbügel (17) teilweise aufgenommen und am Innen-Trennwandteil (20) abgestützt ist, wenn sich der Griffbügel (17) in einer waagerechten Lage befindet.

9. Siebkorb nach einer Kombination der Ansprüche 6 und 7, **dadurch gekennzeichnet, dass** die Trennwand (12) zumindest ein Lateral-Verbindungsteil (24) aufweist, das eines der Innen-Trennwandteile (20) mit einer von mehreren Flächen (3) verbindet, auf denen ein weiterer Siebkorb (9) absetzbar ist.

10. Siebkorb nach Anspruch 4, **dadurch gekennzeichnet, dass** das untere Wandteil (13) eine maschinenlesbare Markierung (29) aufweist, die insbesondere als Muster ausgeführt ist.

11. Siebkorb nach einem der vorhergehenden Ansprüche, **dadurch gekennzeichnet, dass** außerhalb des Schutzraums (11) eine maschinenlesbare Schnittstelle (5) zur Erkennung einer Position des Siebkorbs (9) vorgesehen ist.

12. Verfahren zum Greifen eines Siebkorbs (9) innerhalb eines Behälters (10), wobei der Siebkorb (9) nach einem der vorhergehenden Ansprüche ausgeführt ist und wobei ein Arm (22) des Greifers (25) von oben in den Schutzraum (11) und unter das Abstützteil bewegt wird und anschließend zum Herausheben des Siebkorbs (9) angehoben wird.

13. Verfahren nach Anspruch 12, **dadurch gekennzeichnet, dass** der Greifer (25) zunächst vertikal nach unten und anschließend horizontal unter das Abstützteil bewegt wird.

14. Verfahren nach einem der Ansprüche 12 oder 13, **dadurch gekennzeichnet, dass** von einer mit dem Greifer (25) gekoppelten optischen Erfassungseinrichtung (28) ein Bereich innerhalb des Schutzraumes (11) erfasst wird, in dem sich eine maschinenlesbare Markierung (29) befindet, wobei ein Fehler an eine Steuerungseinrichtung (8) gemeldet wird, wenn die maschinenlesbare Markierung (29) nicht oder nicht ausreichend erfasst werden kann.

15. Verfahren nach Anspruch 14, **dadurch gekennzeichnet, dass** die Steuerungseinrichtung (8) den Greifer (25) nach der Meldung des Fehlers veranlasst,
- sich nicht in den Schutzraum (11) zu bewegen oder
- eine Bewegung in Richtung auf den Schutzraum (11) zu stoppen oder
- sich wieder aus dem Schutzraum (11) herauszubewegen.

16. System, **dadurch gekennzeichnet, dass** das System den Siebkorb (9) und das Siebkorbpositionierelement (1) nach einem der Ansprüche 1 bis 11 und einen Behälter (10) umfasst, wobei der Behälter (10) eine Wanne zur Aufnahme des Siebkorbs (9) umfasst, wobei der Siebkorb (9) mit dem Siebkorbpositionierelement (1) in die Wanne des Behälters (10) einsetzbar ist und derart angepasst ist, dass die mechanische Schnittstelle (2) zur Einschränkung der Bewegung des Siebkorbs (9) im Behälter (10) an einer Innenwand (11) der Wanne anlegbar ist.
